# EUROPEAN PATENT SPECIFICATION

(11) **EP 1 612 284 B1**
(45) Date of publication and mention of the grant of the patent: **21.03.2007**
(21) Application number: 05013918.7
(22) Date of filing: 28.06.2005
(51) Int. Cl.: C12Q 1/70

(54) **Method of detecting and quantifying cytomegalovirus**
Verfahren zur Detektion und Quantifizierung von Cytomegalovirus
Méthode de détection et de quantification du cytomégalovirus

(30) Priority: 30.06.2004 JP 2004194367
(43) Date of publication of application: 04.01.2006
(73) Proprietor: TOSOH CORPORATION, Shunan-shi Yamaguchi-ken 746-8501 (JP)
(72) Inventor: Ohnaka, Satoru, Machida-shi, Tokyo (JP); Hayashi, Toshinori, Sagamihara-shi, Kanagawa-ken (JP)
(74) Representative: Pautex Schneider, Nicole Véronique

(56) References cited:
- EP-A- 0 803 578
- EP-A- 0 969 101
- WO-A-98/38339
- US-A- 5 783 383
- MURPHY EAIN ET AL: "Coding potential of laboratory and clinical strains of human cytomegalovirus." PROCEEDINGS OF THE NATIONAL ACADEMY OF SCIENCES OF THE UNITED STATES OF AMERICA, vol. 100, no. 25, 9 December 2003 (2003-12-09), pages 14976-14981, XP002348007 ISSN: 0027-8424
- MCSHARRY BRIAN P ET AL: "The most abundantly transcribed human cytomegalovirus gene (beta2.7) is non-essential for growth in vitro." JOURNAL OF GENERAL VIROLOGY, vol. 84, no. 9, September 2003 (2003-09), pages 2511-2516, XP002348008 ISSN: 0022-1317

## Description

The present invention relates to a method of detecting and quantifying CMV using specific nucleic acid sequences.

Cytomegalovirus is an icosahedral DNA virus in the β human herpesvirus family. 90% of adults in Japan are infected with CMV. CMV infections are usually caught postnatally and are often latent infections without particular disease manifestation. Acquired infections are transmitted contagiously from urine, saliva, breast milk and semen or iatrogenically by blood transfusion and organ transplantation. CMV is characterized in that it remains dormant within the body for life until it activates from the dormant state (reactivation), triggered by a physiological change in the host, and causes recurrent outbreaks. In patients with malignant tumors or AIDS or organ transplant recipients, immunodeficiency tends to trigger reactivation of CMV which leads to pneumonitis, hepatitis, retinitis, encephalitis and nephritis. Especially, deaths from pneumonitis and the like after successful organ transplantation are a big problem. Therefore, establishment of fast and sensitive diagnoses of reactivated CMV infection which can cause fetal diseases is important for early treatment.

Conventional diagnosis of CMV infection requires identification of CMV isolated from the body fluid of patients which takes 2 to 3 weeks and are not speedy. Serologic diagnosis has a problem of non-specific reactions of antibodies and drawbacks such as inadequate sensitivity and difficulty in virus monitoring by change in antibody titer.

A real-time PCR (polymerase chain reaction) assay for detection of DNA derived from CMV has been developed recently. It uses primers specific for DNA derived from CMV to amplify the virus DNA by PCR and detect the DNA and is characterized by its high sensitivity. However, because of the ability of CMV to establish a dormant infection in various organs, reactivated infection and dormant infection cannot be distinguished just by PCR-based DNA detection. An RT-RCR (reverse transcriptase-polymerase chain reaction) assay has also been proposed to amplify and detect mRNA expressed by reactivated CMV. However, it requires a step of converting mRNA into cDNA and manipulative skills and takes a long time to give results. In addition, if a trace amount of DNA is present, it may give positive results even in the absence of mRNA.

On the other hand, some methods for amplification and detection of RNA which give RNA as the amplification product with no need for heating or cooling of reaction solutions, unlike RT-PCT in which cDNA reverse-transcript from RNA is amplified by heating and cooling the reaction solution, have been reported (for example, NASBA disclosed in JP-A-2-5864, TMA disclosed in JP-A-4-500 759 and the amplification and detection method disclosed in JP-A-2000-014400). These methods involve synthesis of double-stranded DNA having a promoter sequence for an RNA polymerase, which is transcribed into an RNA transcript having a specific sequence.

More specifically, in the case of an arbitrary RNA, (1) an RNA-dependent DNA polymerase (reverse transcriptase) synthesizes the DNA complementary to a specific sequence in the RNA which distinguishes it from other RNAs by using a DNA primer complementary to a 3' region of the specific sequence and the RNA as the template, (2) an enzyme having a ribonuclease H activity degrades the RNA strand in the RNA-DNA double strand produced by the reverse transcription to leave a single-stranded DNA behind, (3) a DNA-dependent DNA polymerase synthesizes a double-stranded DNA having a promoter sequence for an RNA polymerase by using a DNA primer which is complementary to a 3' region of the single strand and has the above-mentioned promoter sequence at the 5' end, and (4) an RNA polymerase transcribes the double-stranded DNA to give a transcript (an RNA having the specific sequence). The RNA transcript having the specific sequence serves as the template for the above-mentioned reaction (1) and hybridizes with the DNA primer used in the above-mentioned reaction (1) to provoke the reaction (2) and the subsequent reactions, and the chain reaction proceeds to amplify the RNA.

These methods are characterized in that it is not necessary to heat and cool the reaction solution as in PCR or carry out RNA reverse transcription and the subsequent amplification separately. The method disclosed in JP-A-9-289900 is an example of application of the method disclosed in JP-A-2-5864 to amplification and detection of an mRNA derived from CMV. However, this method involves trapping of the amplified RNA by a capture probe adsorbed or bound to a solid support, hybridization with a detection probe, separation of the unreacted detection probe and detection of the complex consisting of the capture probe, the amplified RNA and the detection probe, and, therefore, is far from speedy. In this respect, the amplification and detection method disclosed in JP-A-2000-014400 which amplifies a specific sequence or a sequence complementary to the specific sequence and monitors the amplification simultaneously in the presence of a fluorescent intercalative dye-labeled oligonucleotide probe hybridizable with the amplified specific sequence (disclosed in Japanese patent application 2000-154431) can be carried out isothermally in one step and is a speedy quantification method.

However, no probes or primers have been known to be suitable for detection and amplification of an mRNA derived from CMV by the method disclosed in JP-A-2000-014400, because the higher order structure of the target mRNA seems to hinder the oligonucleotide from binding and, as a result, lower the efficiency of the isothermal amplification and detection of mRNA in the method disclosed in JP-A-2000-014400. Therefore, oligonucleotides which enables isothermal amplification and detection of mRNA without reduction in binding efficiency have been demanded.

The object of the present invention is to provide a fast method for detecting and quantifying reactivated CMV isothermally in one step by applying the method disclosed in JP-A-2000-014400 to amplification and detection of an mRNA derived from CMV in a sample obtained from cells infected with CMV.

The present invention has been accomplished to attain the above-mentioned object. The invention defined in Claim 1 of the present application provides a method of amplifying an mRNA derived from cytomegalovirus (hereinafter referred to as CMV) in a sample obtained from cells infected with CMV by using a first primer complementary to part of a specific sequence in the mRNA of the β2.7 gene derived from CMV (which is homologous or complementary to at least 10 consecutive bases in SEQ ID NO:6, 7 or 8) and a second primer homologous to part of the specific sequence (which is homologous or complementary to at least 10 consecutive bases in SEQ ID NO:3, 4 or 5) (either of which additionally has a promoter sequence for an RNA polymerase at the 5' end), which comprises (1) synthesizing a cDNA complementary to the specific sequence by the action of an enzyme having an RNA-dependent DNA polymerase activity by using a single-stranded RNA as a template, (2) degrading the RNA in the resulting RNA-DNA double strand by an enzyme having a ribonuclease H activity (to give a single stranded DNA), (3) forming a double-stranded DNA containing a sequence homologous or complementary to the specific sequence and a promoter sequence which can direct RNA transcription by the action of an enzyme having a DNA-dependent DNA polymerase activity by using the single stranded DNA and (4) transcribing the double-stranded DNA into an RNA transcript (which acts as a template in the reaction (1)) by the action of an enzyme having an RNA polymerase activity by using the double stranded DNA as a template.

The invention defined in Claim 2 of the present application provides a method of detecting or quantifying an mRNA derived from CMV, which comprises conducting the amplification as defined in Claim 1 in the presence of an oligonucleotide homologous or complementary to at least 10 consecutive bases in SEQ ID NO:9 or 10 which is labeled with a fluorescent intercalative dye (hereinafter referred to as an oligonucleotide probe) and measuring the fluorescent intensity of the reaction solution, wherein the oligonucleotide probe alters its fluorescence upon hybridization of the oligonucleotide with the RNA transcript.

The invention defined in Claim 3 of the present application provides a amplification method according to Claim 1, wherein an oligonucleotide having a sequence of SEQ ID NO:1 or 2 is used to cleave the mRNA derived from CMV at the 5' end of the specific sequence.

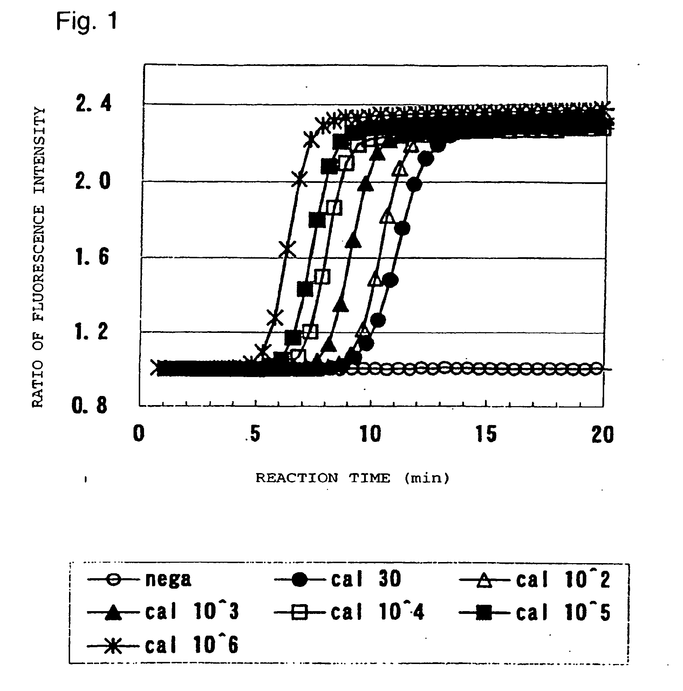

Fig. 1 is a graph showing the fluorescence enhancement which increases with reaction time and RNA synthesis at initial RNA amounts of from 10⁶ copies/30 µl to 30 copies/30 µl. cal30: 30 copies of the β2.7 RNA/5 µL, cal10^2: 10² copies of the β2.7 RNA/5 µL, cal10^3: 10³ copies of the β2.7 RNA/5 µL, cal10^4: 10⁴ copies of the β2.7 RNA/5 µL, cal10^5: 10⁵ copies of the β2.7 RNA/5 µL, cal10^6: 10⁶ copies of the β2.7 RNA/5 µL. For nega, only the RNA diluent was used instead of the RNA sample.

Now, the present invention will be described in detail.

The present invention uses RNA amplification which gives RNA as the amplification product to make it possible to detect an mRNA derived from CMV isothermally in one step quickly. Especially, addition of a fluorescent intercalative dye-labeled oligonucleotide probe hybridizable with the transcript to the reaction solution makes it possible to amplify the specific or complementary sequence and detect (monitor) the way it is amplified. The probe used for monitoring is obtained by liking a fluorescent intercalative dye to an oligonucleotide hybridizable with the specific or complementary sequence so that the fluorescent intercalative dye alters its fluorescence upon hybridization with the transcript. The above-mentioned monitoring is the most suitable mode of carrying the present invention. Though there is no particular restriction on how to link the fluorescent intercalative dye as the label to the oligonucleotide, it is preferred to link the fluorescent intercalative dye to a phosphorus atom via a linker. They may be liked, for example, by introducing a functional group such as an amino group into the oligonucleotide chain or at either end of the oligonucleotide, then optionally introducing a functional group reactive or linkable with the functional group introduced to the oligonucleotide into the fluorescent intercalative dye, and bonding the two functional groups. The details are disclosed in JP-A-2000-154431 or Ishiguro, T (1996) Nucleic Acids Res. 24 (24) 4992-4997.

The present invention is carried out isothermally at relatively low temperatures of from 35°C to 50°C (preferably 44°C).

To carry out the present invention, a specific sequence is an mRNA derived from CMV is first selected. In the present invention, the mRNA of the CMV β2.7 gene is selected as the target mRNA because it is not expressed by dormant CMV but expressed in a large amount by reactivated CMV. Then, the sequences of the first and second primers used in the present invention are designed for the selected specific sequence in the target mRNA. For example, oligonucleotides containing a sequence homologous or complementary to at least 10 consecutive nucleotides in SEQ ID NOS:3 to 8 are preferable examples of the primer used for amplification of the mRNA of the CMV β2.7 gene, and oligonucleotides containing a sequence homologous or complementary to at least 10 consecutive nucleotides in SEQ ID NO:9 or 10 are preferable examples of the detection probe, as described in Examples.

The samples mean mRNA-containing nucleic acid samples. In the present invention, samples prepared from human cells infected with CMV according to the procedure disclosed in JP-A-7-59572, for example, are used, and an mRNA as exemplified above is directly amplified and detected to detect and quantify CMV in the human cells from which the samples are obtained.

The present invention will be described in further detail below. When the mRNA of the CMV β2.7 gene as the amplification and detection target is present in a sample, (1) the first primer (complementary to a 3' region of the specific sequence) hybridizes with the specific sequence in the mRNA and elongates as a cDNA along the specific sequence in the mRNA as the template by the action of an RNA-dependent DNA polymerase to form a RNA-DNA double strand, (2) then, the RNA in the RNA-DNA double strand is degraded by an enzyme having a ribonuclease H activity to leave a single-stranded DNA, (3) the second primer (which is homologous to a 5' region of the target RNA and additionally has a promoter sequence for an RNA polymerase at the 5' end) hybridizes with the single-stranded DNA to direct an enzyme having a DNA-dependent DNA polymerase activity to form a double-stranded DNA having a promoter sequence which is transcribed into an RNA homologous to the target RNA sequence, and (4) the double-stranded DNA is transcribed by an enzyme having an RNA polymerase activity into an RNA transcript homologous to the specific sequence. Since the RNA transcript having the specific sequence serves as the template for the reaction (1), the above-mentioned reactions (1) to (4) proceed sequentially until the enzymes which catalyze RNA and DNA syntheses run out of their substrates or inactivate.

In the above-mentioned embodiment, a second primer additionally having a promoter sequence for the RNA polymerase at the 5' end is used (hereinafter referred to as the first embodiment). In the present invention, a first primer additionally having a promoter sequence for the RNA polymerase at the 5' end may be used (hereinafter referred to as the second embodiment). In the first embodiment of the present invention, in order to improve the sensitivity by amplifying the specific sequence more efficiently, it is preferred to cleave the RNA containing the specific sequence at the 5' end of the specific sequence prior to the above-mentioned reaction (1) (JP-A-2000-014400).

For the RNA cleavage, the enzyme having a ribonuclease H activity, which is used in the subsequent reactions, for example, may be used in the presence of an oligonucleotide (scissor oligonucleotide) complementary to a region of the RNA which flanks the 5' end of the specific sequence with an overlap. As such an oligonucleotide, an oligonucleotide having a base sequence represented by SEQ ID NO:1 or 2 is preferred. Preliminary treatment such as amination of the 3' end of the scissor oligonucleotide is preferred to prevent the oligonucleotide from serving as a primer.

For detection and quantification of the mRNA of the CMV β2.7 gene, it is necessary to check for the presence of the specific sequence by detecting the presence of the amplified specific sequence or to estimate the amount of the specific sequence present in a sample (number of copies of the target RNA) from the amount (number of RNA copies) of the amplified specific sequence. The specific sequence may be detected by sandwich assay with reaction solutions using immobilized and labeled probes hybridizable with the specific sequence after the above-mentioned reactions are performed for a predetermined time. However, it is preferred to use a fluorescent intercalative dye-labeled oligonucleotide probe which is hybridizable with the specific sequence. Since the probe does not interfere with the reactions (1) to (4), it is particularly preferred to amplify the specific sequence in its presence and monitor the amplification of the specific sequence. When the specific sequence is amplified in the presence of a fluorescent intercalative dye-labeled oligonucleotide probe, preliminary addition of glycolic acid, biotin or the like to the 3' end is preferable to prevent the probe from elongating like a primer. During the amplification, the fluorescent signal from the fluorescent intercalative dye-labeled oligonucleotide probe hybridized with the specific sequence is measured with a fluorometer. Comparison of the information given by the fluorescent profile (such as the amplification time required until the fluorescence intensity of the fluorescent dye reaches a certain level) with the information obtained from a fluorescent profile for a known amount of the standard RNA makes it possible to determine the presence or absence of the specific sequence or estimate the amount of the specific sequence present in the sample (number of copies of the target RNA) from the amount (number of RNA copies) of the amplified specific sequence. The oligonucleotide probe preferably has a base sequence consisting of at least 10 consecutive bases in SEQ ID NO:9 or 10.

Assembling a ready-made detection kit greatly facilitates the method of the present invention as described above. The detection kit preferably contains all the reagents necessary for amplification and detection of the specific sequence such as the first and the second primer, the scissor primer (in the case of the first embodiment), the fluorescent intercalative dye-labeled oligonucleotide probe, an enzyme having an RNA-dependent DNA polymerase activity, an enzyme having a ribonuclease H activity, an enzyme having a DNA-dependent DNA polymerase activity, an enzyme having an RNA polymerase activity and the substrates for these enzymes. It is noteworthy that all the reagents can be put in a single vessel. Namely, just dispensing a certain amount of a sample into the single vessel provokes amplification and detection of the mRNA of the CMV β2.7 gene automatically. The only requirement for the vessel is that it is partly transparent so that the signal from the fluorescent dye can be measured from the outside. Vessels which can be sealed after samples are dispensed are particularly preferable to prevent contamination.

As described above, the present invention makes it possible to amplify, detect and quantify the mRNA of the CMV β2.7 gene by carrying out amplification and detection isothermally at relatively low temperatures (from 35°C to 50°C, preferably 44°C) in one step in a short time.

In the present invention, it is possible to determine an initial amount of a target RNA (the mRNA of the CMV β2.7 gene) in a sample in a short time easily by synthesizing a double-stranded DNA having a promoter region for a DNA-dependent DNA polymerase from the target RNA, then synthesizing a large amount of RNA single strands from the double-stranded DNA to increase the single-stranded RNA production exponentially, measuring the fluorescence enhancement resulting from hybridization of a fluorescent intercalative dye-labeled probe with the RNA transcript and analyzing the enhancement of the fluorescence intensity.

The present invention also provides combinations of oligonucleotides used for detection of the mRNA of the CMV β2.7 gene, i.e., combinations of oligonucleotide primers for amplification of the mRNA of the CMV β2.7 gene and oligonucleotides for detection of the mRNA of the CMV β2.7 gene and thereby provides a simple, fast and sensitive method of detecting and quantifying CMV and a detection and quantification kit in the medical field.

Now, the present invention will be described in further detail by referring to Examples. Table 1 shows combinations of the first primer, second primer and scissor primer which may be used. Among them, the combination which allowed the most efficient amplification, No.9, will be described in these Examples. However, the present invention is by no means restricted to these specific Examples.

**Table 1**

| Combination | First primer | Second primer | Scissor primer | Length of amplification product (mer) |
|---|---|---|---|---|
| 1 | SEQ ID NO 6 | SEQ ID NO:3 | SEQ ID NO:1 | 123 |
| 2 | SEQ ID NO:7 | SEQ ID NO:3 | SEQ ID NO:1 | 129 |
| 3 | SEQ ID NO:8 | SEQ ID NO:3 | SEQ ID NO:1 | 142 |
| 4 | SEQ ID NO:6 | SEQ ID NO:4 | SEQ ID NO:1 | 123 |
| 5 | SEQ ID NO:7 | SEQ ID NO:4 | SEQ ID NO:1 | 129 |
| 6 | SEQ ID NO:8 | SEQ ID NO:4 | SEQ ID NO:1 | 142 |
| 7 | SEQ ID NO:6 | SEQ ID NO:5 | SEQ ID NO:2 | 120 |
| 8 | SEQ ID NO:7 | SEQ ID NO:5 | SEQ ID NO:2 | 126 |
| 9 | SEQ ID NO:8 | SEQ ID NO:5 | SEQ ID NO:2 | 139 |

### EXAMPLE 1

A fluorescent intercalative dye-labeled oligonucleotide probe was prepared.

Oxazole yellow, a conventional fluorescent intercalative dye, was attached as the label to the phosphorus atom between the 4th nucleotide (G) and the 5th nucleotide (C) from the 5' end of the sequence shown in SEQ ID NO:9 to give an oxazole yellow-labeled oligonucleotide probe (SEQ ID NO:9) (Ishiguro, T (1996) Nucleic Acids Res. 24 (24) 4992-4997).

### EXAMPLE 2

The β2.7 RNA at various initial copy numbers was detected using a combination of oligonucleotide primers of the present invention.
(1) The β2.7 RNA was prepared.
   The β2.7 RNA was prepared by cloning and in vitro transcription of a 2154-bp double-stranded DNA within the nucleotide sequence of the CMV β2.7 gene (2471 bases from the 184889th base to the 187359th base of National Center Biotechnology Information, accession No.: X17403) and isolated.
   The β2.7 RNA (2154-mer) as the sample was quantified by UV absorptionmetry at 260 nm and diluted to 1.0×10⁶ copies/5 µl to 30 copies/5 µl with an RNA diluent (10 mM Tris-HCl (pH 8.0), 0.1 mM EDTA, 0.5 U/µl RNase inhibitor, 5.0 mM DTT). As the control (nega), the diluent was used.
(2) 20.0 µl portions of a reaction solution of the following composition were dispensed into 0.5 ml PCR tubes (Gene Amp Thin-Walled Reaction Tubes, Perkin Elmer), and 5 µl of the above-mentioned RNA sample was added. The composition of the reaction solution (in terms of the final concentration in the reaction solutions after the addition of an enzyme solution)
   60.0 mM Tris-HCl buffer (pH 8.6)
   18.0 mM Magnesium chloride
   100.0 mM Potassium chloride
   1.0 mM DTT
   0.25 mM dATP, dCTP, dGTP, dTTP
   3.0 mM ATP, CTP, UTP
   2.25 mM GTP
   3.6 mM ITP
   1.0 µM each of a first oligonucleotide primer (SEQ ID NO:8) and a second oligonucleotide primer (SEQ ID NO:5) (the second primer additionally had SEQ ID NO:11 (the promoter sequence for T7 polymerase) at the 5' end)
   0.16 µM a scissor oligonucleotide (SEQ ID NO:2; for cleavage of the β2.7 RNA at the site of hybridization with the second primer and having an aminated 3' end)
   6 U Ribonuclease inhibitor (Takara Bio)
   13.0% DMSO
   Distilled water for volume adjustment
   12.0 nM fluorescent intercalative dye-labeld oligonucleotide probe (prepared in Example 1)
(3) The reaction solutions were incubated at 43°C for 5 minutes, and 5.0 µl of an enzyme solution of the following composition preincubated at 43°C for 2 minutes was added.
   The composition of the enzyme solution (in terms of the final concentration at the time of the reaction)
   2.0% Sorbitol
   6.4 U AMV reverse transcriptase (Takara Bio)
   142 U T7 RNA polymerase (GIBCO)
   3.6 µg Bovine serum albumin
   Distilled water for volume adjustment
(4) The fluorescence intensities of the reaction solutions in the PCR tubes were directly monitored at 43°C in a thermostatic fluorescent spectrophotometer at an excitation wavelength of 470 nm and an emission wavelength of 510 nm.

Fig. 1 shows the time courses of the ratio of fluorescence intensities of the samples (fluorescence intensity at a certain time/background fluorescence intensity) from addition of the enzyme solution at 0 minute at the initial amounts of the RNA of from 1×10⁶ copies/30 µl to 30 copies/30 µl. The fluorescence profiles were dependent on the initial β2.7 RNA concentrations as shown in Fig. 1 and suggest that it is possible to quantify the mRNA of the CMV β2.7 gene in an unknown sample.

## Claims

1. A method of amplifying an mRNA derived from cytomegalovirus (hereinafter referred to as CMV) in a sample by using a first primer complementary to part of a specific sequence in the mRNA derived from CMV and a second primer homologous to part of the specific sequence (either of which additionally has a promoter sequence for an RNA polymerase at the 5' end), which comprises (1) synthesizing a cDNA complementary to the specific sequence by the action of an enzyme having an RNA-dependent DNA polymerase activity by using a single-stranded RNA as a template, (2) degrading the RNA in the resulting RNA-DNA double strand by an enzyme having a ribonuclease H activity (to give a single stranded DNA), (3) forming a double-stranded DNA containing a sequence homologous or complementary to the specific sequence and a promoter sequence which can direct RNA transcription by the action of an enzyme having a DNA-dependent DNA polymerase activity by using the single stranded DNA and (4) transcribing the double-stranded DNA into an RNA transcript (which acts as a template in the reaction (1)) by the action of an enzyme having an RNA polymerase activity by using the double stranded DNA as a template, wherein the mRNA is the mRNA of the CMV β2.7 gene, the first primer is an oligonucleotide homologous or complementary to at least 10 consecutive bases in SEQ ID NO:6, 7 or 8, and the second primer is an oligonucleotide homologous or complementary to at least 10 consecutive bases in SEQ ID NO:3, 4 or 5.

2. A method of detecting or quantifying an mRNA derived from CMV, which comprises conducting the amplification as defined in Claim 1 in the presence of an oligonucleotide homologous or complementary to at least 10 consecutive bases in SEQ ID NO:9 or 10 which is labeled with a fluorescent intercalative dye (hereinafter referred to as an oligonucleotide probe) and measuring the fluorescent intensity of the reaction solution, wherein the oligonucleotide probe alters its fluorescence upon hybridization of the oligonucleotide with the RNA transcript.

3. The method of amplifying an mRNA according to Claim 1, which is carried out in the presence of an oligonucleotide complementary to a region of the mRNA of the CMV β2.7 gene which flanks the 5' end of the specific sequence with an overlap with the specific sequence when the second primer has a promoter sequence, wherein the oligonucleotide has a sequence of SEQ ID NO:1 or 2.

## Patentansprüche

1. Verfahren zum Amplifizieren einer von dem Zytomegalie-Virus (nachstehend als CMV bezeichnet) stammenden mRNA in einer Probe unter Verwendung eines ersten Primers, der zu einem Teil einer spezifischen Sequenz in der von dem CMV stammenden mRNA komplementär ist, und eines zweiten Primers, der zu einem Teil der spezifischen Sequenz homolog ist (von denen einer zusätzlich eine Promotorsequenz für eine RNA-Polymerase am 5'-Ende hat), mit den folgenden Schritten:
(1) Synthetisieren einer cDNA, die zu der spezifischen Sequenz komplementär ist, durch die Wirkung eines Enzyms mit einer RNA-abhängige-DNA-Polymerase-Aktivität unter Verwendung einer einzelsträngigen RNA als Template;
(2) Abbauen der RNA in dem resultierenden RNA-DNA-Doppelstrang mit einem Enzym mit einer Ribonuclease-H-Aktivität (sodass eine einzelsträngige DNA entsteht);
(3) Herstellen einer doppelsträngigen DNA, die eine Sequenz, die zu der spezifischen Sequenz homolog oder komplementär ist, und eine Promotorsequenz enthält, die durch die Wirkung eines Enzyms mit einer DNA-abhängige-Polymerase-Aktivität unter Verwendung der einzelsträngigen DNA eine RNA-Transkription befehlen kann; und
(4) Transkribieren der doppelsträngigen DNA zu einem RNA-Transkript (das als Template in der Reaktion (1) fungiert) durch die Wirkung eines Enzyms mit einer RNA-Polymerase-Aktivität unter Verwendung der doppelsträngigen DNA als Template,
wobei die mRNA die mRNA des CMV-β2.7-Gens ist, der erste Primer ein Oligonucleotid ist, das zu mindestens 10 aufeinanderfolgenden Basen in der SEQ ID NO: 6, 7 oder 8 homolog oder komplementär ist, und der zweite Primer ein Oligonucleotid ist, das zu mindestens 10 aufeinanderfolgenden Basen der SEQ ID NO: 3, 4 oder 5 homolog oder komplementär ist.

2. Verfahren zum Nachweisen oder zur Quantitätsbestimmung einer von dem CMV stammenden mRNA, das das Durchführen der Amplifikation nach Anspruch 1 in Gegenwart eines Oligonucleotids, das zu mindestens 10 aufeinanderfolgenden Basen in der SEQ ID NO: 9 oder 10 homolog oder komplementär ist und mit einem interkalierenden Fluoreszenzfarbstoff markiert ist (nachstehend als Oligonucleotid-Probe bezeichnet), und das Messen der Fluoreszenzintensität der Reaktionslösung aufweist, wobei die Oligonucleotid-Probe ihre Fluoreszenz nach Hybridisierung des Oligonucleotids mir dem RNA-Transkript ändert.

3. Verfahren zum Amplifizieren einer mRNA nach Anspruch 1, das in Gegenwart eines Oligonucleotids durchgeführt wird, das zu einer Region der mRNA des CMV-β2.7-Gens komplementär ist, die das 5'-Ende der spezifischen Sequenz mit einer Überlappung mit der spezifischen Sequenz flankiert, wenn der zweite Primer eine Promotorsequenz hat, wobei das Oligonucleotid eine Sequenz der SEQ ID NO: 1 oder 2 hat.

## Revendications

1. Procédé d'amplification d'un ARNm obtenu à partir du cytomégalovirus (appelé ci-après virus CMV) dans un échantillon en utilisant une première amorce complémentaire d'une partie d'une séquence spécifique dans l'ARNm obtenu à partir du cytomégalovirus et une seconde amorce homologue à une partie de la séquence spécifique (dont l'une ou l'autre comporte en outre une séquence d'activateur pour une polymérase d'ARN à extrémité 5'), qui comprend : (1) la synthèse d'un ADNc complémentaire de la séquence spécifique au moyen de l'action d'une enzyme ayant une activité de polymérase d'ADN dépendant de l'ARN en utilisant un ARN à un seul brin en tant qu'agent structurant, (2) la dégradation de l'ARN dans le double brin d'ARN-ADN résultant par une enzyme ayant une activité de ribonucléase H (pour donner un ADN à un seul brin), (3) la formation d'un ADN à double brin contenant une séquence homologue à la séquence spécifique, ou complémentaire de celle-ci, et une séquence d'activateur qui peut diriger la transcription d'ARN directe au moyen de l'action d'une enzyme présentant une activité de polymérase d'ADN dépendant de l'ADN en utilisant l'ADN à un seul brin, et (4) la transcription de l'ADN à double brin en un produit de transcription d'ARN (qui agit en tant qu'agent structurant dans la réaction (1)) au moyen de l'action d'une enzyme présentant une activité de polymérase d'ARN en utilisant l'ADN à double brin en tant d'agent structurant où l'ARNm est l'ARNm du gène β2.7 du cytomégalovirus, la première amorce est un oligonucléotide homologue ou complémentaire à au moins 10 bases consécutives dans les séquences à numéro d'identification: 6, 7 ou 8, et la seconde amorce est un oligonucléotide homologue ou complémentaire à au moins 10 bases consécutives dans les séquences à numéro d'identification : 3, 4 ou 5.

2. Procédé de détection ou de quantification d'un ARNm obtenu à partir du cytomégalovirus, qui comprend l'exécution de l'amplification comme défini dans la revendication 1 en la présence d'un oligonucléotide homologue ou complémentaire à au moins 10 bases consécutives dans la séquence à numéro d'identification : 9 ou 10 qui a été marquée avec un colorant intercalaire fluorescent (appelé ci-après sonde à oligonucléotide) et la mesure de l'intensité de fluorescence de la solution de réaction, où la sonde à oligonucléotide modifie sa fluorescence lors de l'hybridation de l'oligonucléotide avec le produit de transcription d'ARN.

3. Procédé d'amplification d'un ARNm selon la revendication 1, qui est exécuté en la présence d'un oligonucléotide complémentaire d'une région de l'ARNm du gène β2.7 du cytomégalovirus qui encadre l'extrémité 5' de la séquence spécifique avec un chevauchement avec la séquence spécifique lorsque la seconde amorce comporte une séquence d'activateur, où l'oligonucléotide comporte une séquence à numéro d'identification : 1 ou 2.


### SEQUENCE LISTING

<110> TOSOH Corporation
<120> METHOD OF DETECTING AND QUANTIFYING CYTOMEGALOVIRUS
<130> PA211-1465
<160> 11
<170> PatentIn version 3.2
<210> 1
   <211> 22
   <212> DNA
   <213> Artificial
<220>
<223> CBS3
<400> 1
   gattaaggaa aggagaagat aa 22
<210> 2
   <211> 20
   <212> DNA
   <213> Artificial
<220>
   <223> CBS32
<400> 2
   ccgagattaa ggaaaggaga 20
<210> 3
   <211> 23
   <212> DNA
   <213> Artificial
<220>
   <223> CBF3
<400> 3
   atctcggatt atcatttccc tct 23
<210> 4
   <211> 21
   <212> DNA
   <213> Artificial
<220>
   <223> CBF5
<400> 4
   atctcggatt atcatttccc t 21
<210> 5
   <211> 23
   <212> DNA
   <213> Artificial
<220>
   <223> CBF4
<400> 5
   tcggattatc atttccctct cct 23
<210> 6
   <211> 17
   <212> DNA
   <213> Artificial
<220>
   <223> CBR1
<400> 6
   tttgaattct tcgagtt 17
<210> 7
   <211> 17
   <212> DNA
   <213> Artificial
<220>
   <223> CBR2
<400> 7
   gtcgactttg aattctt 17
<210> 8
   <211> 16
   <212> DNA
   <213> Artificial
<220>
   <223> CBR3
<400> 8
   cttgttggga atcgtc 16
<210> 9
   <211> 18
   <212> DNA
   <213> Artificial
<220>
   <223> CBINAF1
<400> 9
   tctgcgattc gtggtagg 18
<210> 10
   <211> 20
   <212> DNA
   <213> Artificial
<220>
   <223> CBINAF2
<400> 10
   ctttttaccc tcttgtttat 20
<210> 11
   <211> 28
   <212> DNA
   <213> Artificial
<220>
   <223> T7
<400> 11
   aattctaata cgactcacta tagggaga 28

### SEQUENCE LISTING

<110> TOSOH Corporation
<120> METHOD OF DETECTING AND QUANTIFYING CYTOMEGALOVIRUS
<130> PA211-1465
<160> 11
<170> Patentln version 3.2
<210> 1
   <211> 22
   <212> DNA
   <213> Artificial
<220>
   <223> CBS3
<400> 1
   gattaaggaa aggagaagat aa **2**2
<210> 2
   <211> 20
   <212> DNA
   <213> Artificial
<220>
   <223> CBS32
<400> 2
   ccgagattaa ggaaaggaga **2**0
<210> 3
   <211> 23
   <212> DNA
   <213> Artificial
<220>
   <223> CBF3
<400> 3
   atctcggatt atcatttccc tct 23
<210> 4
   <211> 21
   <212> DNA
   <213> Artificial
<220>
   <223> CBF5
   <400> 4
   atctcggatt atcatttccc t 21
<210> 5
   <211> 23
   <212> DNA
   <213> Artificial
<220>
   <223> CBF4
<400> 5
   tcggattatc atttccctct cct 23
<210> 6
   <211> 17
   <212> DNA
   <213> Artificial
<220>
   <223> CBR1
<400> 6
   tttgaattct tcgagtt 17
<210> 7
   <211> 17
   <212> DNA
   <213> Artificial
<220>
   <223> CBR2
<400> 7
   gtcgactttg aattctt 17
<210> 8
   <211> 16
   <212> DNA
   <213> Artificial
<220>
   <223> CBR3
<400> 8
   cttgttggga atcgtc 16
<210> 9
   <211> 18
   <212> DNA
   <213> Artificial
<220>
   <223> CBINAF1
<400> 9
   tctgcgattc gtggtagg 18
<210> 10
   <211> 20
   <212> DNA
   <213> Artificial
<220>
   <223> CBINAF2
<400> 10
   ctttttaccc tcttgtttat 20
<210> 11
   <211> 28
   <212> DNA
   <213> Artificial
<220>
   <223> T7
<400> 11
   aattctaata cgactcacta tagggaga 28